# EUROPEAN PATENT APPLICATION

(11) **EP 4 239 063 A1**
(43) Date of publication of application: **06.09.2023**
(21) Application number: 22159636.4
(22) Date of filing: 02.03.2022
(51) Int. Cl.: C12N 5/10

(54) **IMPROVED CELL LINES AND METHODS FOR THE PRODUCTION OF ADENO-ASSOCIATED VECTORS**

(71) Applicant: CEVEC Pharmaceuticals GmbH, 51105 Köln (DE)
(72) Inventor: HUSSONG, Michelle, 50858 Köln (DE); BONIFERT, Tobias, 51103 Köln (DE)
(74) Representative: Müller-Boré & Partner Patentanwälte PartG mbB

(57) **Abstract**

The present invention relates to Adeno-associated virus (AAV) producer cell lines in which DNA fragmentation caused by programmed cell death is inhibited, uses of cell lines in which DNA fragmentation caused by programmed cell death is inhibited for the production of AAV, related methods of producing AAV, and methods of producing AAV, comprising the step of exposing the cells in which AAV is produced to an inhibitor of DNA fragmentation caused by programmed cell death during the AAV production phase.

## Description

The present invention relates to Adeno-associated virus (AAV) producer cell lines in which DNA fragmentation caused by programmed cell death is inhibited, uses of cell lines in which DNA fragmentation caused by programmed cell death is inhibited for the production of AAV, related methods of producing AAV, and methods of producing AAV, comprising the step of exposing the cells in which AAV is produced to an inhibitor of DNA fragmentation caused by programmed cell death during the AAV production phase.

In recent years, a rapid increase in the number of gene therapy trials and products based on Adeno-associated virus (AAV)-derived vectors could be observed. Advantages of AAV vectors in gene therapy include an advantageous safety profile, the fact that AAV is not pathogenic, *i.e.,* is not associated with any disease, the stable expression of transgenes, and the possibility of transducing dividing as well as non-dividing cells. However, exhaustive quality control (QC) must be performed on all AAV-derived products to ensure their efficiency and safety.

A major challenge for the production of AAV-based preparations is the identification, characterization, and control of process- and product-related impurities that may occur in the already purified products. Impurities that remain after vector purification include residual amounts of chemical components, proteins and nucleic acids derived from the cell culture system in which the vector product was generated. Nucleic acid impurities include residual DNA, specifically host cell DNA and/or plasmid DNA, wherein residual host cell DNA may exist in two forms: (1) as nuclease-sensitive process-related contamination, *i.e.,* nonspecifically co-purified with the desired AAV vector product; and (2) as nuclease-resistant product-related contamination, *i.e*., encapsulated in AAV particles. This latter type of product impurity cannot be removed by conventional "downstream process" methods.

Residual DNA impurities pose a significant safety hazard because the DNA might encode proteins or regulatory RNAs and even trigger immune toxicity themselves' via TLR9 activation. Thus, e.g., the Food and Drug Administration (FDA) recommendations are that the level of residual cell-substrate DNA should be below 10 ng per dose and a median DNA size of 200 bp or lower.

The encapsulation of fragments of mammalian producer cell genomic DNA has been reported to be generated at a frequency of 1% to 3% of AAV genome-containing particles. However, the mechanism of the packaging of fragments of host cell DNA within AAV particles is not yet understood.

Based on close similarity with the desired vector product, it is difficult to eliminate AAV packaged host cell DNA impurities by vector purification methods. Gradient centrifugation can remove AAV packaged nucleic acid impurities that differ significantly in length from the vector genome. However, there is so far no general technical solution to avoid, reduce and/or remove nuclease-resistant product-related nucleic acid impurities.

Accordingly, the technical problem underlying the present invention is the provision of means for the avoidance, reduction and/or removal of nuclease-resistant product-related nucleic acid impurities.

The solution to the above technical problem is achieved by the embodiments characterized in the claims.

In particular, in a first aspect, the present invention relates to an AAV producer cell line in which DNA fragmentation caused by programmed cell death is inhibited.

In a second aspect, the present invention relates to the use of a cell line in which DNA fragmentation caused by programmed cell death is inhibited for the production of Adeno-associated virus (AAV).

In a preferred embodiment, the cell line is an AAV producer cell line.

The production of recombinant AAV *inter alia* requires the expression of AAV Rep and Cap proteins, usually encoded by the AAV genome, for production of recombinant virus supplied *in trans.* Further, helper genes are used which can be derived from different helper viruses, the most common being helper virus genes taken from Adenovirus (AV), such as E1A, E1B, E2A, E4orf6, or VA RNA. Furthermore, a transfer vector containing the gene of interest (GOI) flanked by AAV inverted terminal repeat sequences (ITRs) is needed.

The most common system for the production of recombinant AAV relies on the introduction of all genes necessary for AAV production into the production cells by transient transfection. Transient transfection usually requires a two- or threeplasmid system: transfer vector containing gene of interest; pHelper with adenoviral helper functions; and pAAV-Rep2CapX (CapX = capsid function of different AAV serotypes) supplying the capsid and replicase functions.

Alternatively, some or all of the genes necessary for AAV production can be stably integrated into the host cell genome.

Accordingly, the term "AAV producer cell line" as used herein relates to any cell line in which some or all of the genes encoding the components necessary for the production of AAV are either transiently expressed in said cell line or are stably integrated into the cell genome.

Preferably, the genes encoding the components necessary for the production of AAV are selected from the group consisting of genes encoding the AAV Cap proteins VP1, VP2, and VP3; genes encoding the AAV Rep proteins Rep78, Rep68, Rep52, and Rep40; genes encoding the adenoviral helper functions E4orf6, E2A and VA-RNA; genes encoding the Ad5 helper genes E1A and E1B; and the gene of interest (GOI) flanked by AAV inverted terminal repeat sequences (ITRs). More preferably, the genes encoding the components necessary for the production of AAV include the genes encoding the AAV Cap proteins VP1, VP2, and VP3; a gene encoding the AAV Rep protein Rep78 or Rep68, a gene encoding the AAV Rep protein Rep52 or Rep40; a gene encoding the adenoviral helper function E4orf6, and the gene of interest (GOI) flanked by AAV ITRs. In specific embodiments, the genes encoding the components necessary for the production of AAV additionally include at least 1, at least 2, at least 3, at least 4, at least 5, or all of the genes encoding the AAV Rep protein Rep78, Rep68, Rep52 and Rep40; and the genes encoding the adenoviral helper functions E2A and VA-RNA; and the genes encoding the Ad5 helper genes E1A and E1B.

Thus, in specific embodiments, in the AAV producer cell lines according to the present invention, at least 1, at least 2, at least 3, at least 4, at least 5, at least 6, at least 7, at least 8, at least 9, at least 10, at least 11, or all of the following genes are transiently expressed in said cell lines or are stably integrated into the cell genome:
the genes encoding the AAV Cap proteins VP1, VP2, and VP3;
a gene or genes encoding either one or both of the AAV Rep proteins Rep78 or Rep68;
a gene or genes encoding either one or both of the AAV Rep proteins Rep52 or Rep40;
the gene of interest (GOI) flanked by AAV ITRs; and
optionally, a gene or genes encoding either one, either two, either three, or all of the adenoviral helper functions E1A, E1B, E4orf6 and E2A.

In related specific embodiments, in the AAV producer cell lines according to the present invention, the following genes are transiently expressed in said cell lines or are stably integrated into the host cell genome:
(i) the genes encoding the AAV Cap proteins VP1, VP2, and VP3; a gene encoding the AAV Rep protein Rep78 or Rep68, a gene encoding the AAV Rep protein Rep52 or Rep40; and the gene of interest (GOI) flanked by AAV ITRs; or
(ii) the genes encoding the AAV Cap proteins VP1, VP2, and VP3; a gene encoding the AAV Rep protein Rep78 or Rep68, a gene encoding the AAV Rep protein Rep52 or Rep40; a gene encoding the adenoviral helper function E4orf6, and the gene of interest (GOI) flanked by AAV ITRs; or
(iii) the genes encoding the AAV Cap proteins VP1, VP2, and VP3; a gene encoding the AAV Rep protein Rep78 or Rep68, a gene encoding the AAV Rep protein Rep52 or Rep40; genes encoding the adenoviral helper functions E4orf6 and E2A, and the gene of interest (GOI) flanked by AAV ITRs; or
(iv) the genes encoding the AAV Cap proteins VP1, VP2, and VP3; a gene encoding the AAV Rep protein Rep78 or Rep68, a gene encoding the AAV Rep protein Rep52 or Rep40; genes encoding the adenoviral helper functions E1A, E1B, E4orf6 and E2A, and the gene of interest (GOI) flanked by AAV ITRs; or
(v) the genes encoding the AAV Cap proteins VP1, VP2, and VP3; the genes encoding the AAV Rep proteins Rep78, Rep68, Rep52 and Rep40; the genes encoding the adenoviral helper functions E4orf6 and E2A; and the gene of interest (GOI) flanked by AAV ITRs; or
(vi) the genes encoding the AAV Cap proteins VP1, VP2, and VP3; the genes encoding the AAV Rep proteins Rep78, Rep68, Rep52 and Rep40; the genes encoding the adenoviral helper functions E1A, E1B, E4orf6 and E2A; and the gene of interest (GOI) flanked by AAV ITRs.

AAV in the present invention is not limited to particular AAV serotypes. Thus, AAV can be selected from the group consisting of AAV serotype 1 (AAV1), AAV2, AAV3, AAV4, AAV5, AAV6, AAV7, AAV8, AAV9, AAV10, AAV11, AAVDJ, AAVDJ8, AAVrh10, hybrids of two or more different of said serotypes, and said serotypes having mutations that alter the tropism of the AAV serotype. Preferably, AAV is selected from the group consisting of AAV2, AAV5, AAV6, AAV8, and AAV9, wherein AAV8 is particularly preferred.

The AAV producer cell lines according to the present invention can be derived from any suitable cell line known in the art. However, in preferred embodiments, said cell lines are derived from a cell line, selected from the group consisting of CAP cells, AGE1.hn, HEK293, PER.C6, NSO1, COS-7, BHK, CHO, Sf9, S2, CV1, VERO, HeLa, MDCK, BRL3A, W138, and HepG2 cells, wherein CAP cells or HEK293 cells are particularly preferred.

According to the present invention as defined in the above first and second aspects, encapsulation of host cell DNA is inhibited by inhibiting DNA fragmentation caused by programmed cell death in the cells used for AAV production.

As used herein, the term "programmed cell death" includes apoptosis, parthanatos, and pyroptosis. Accordingly, the term "DNA fragmentation caused by programmed cell death" as used herein includes apoptotic DNA fragmentation, parthanatotic DNA fragmentation, and pyroptotic DNA fragmentation. In this context, apoptotic DNA fragmentation is of particular relevance in the present invention.

Preferably, inhibition of DNA fragmentation caused by programmed cell death encompasses any direct or indirect inhibition of endonucleases. For example, direct inhibition of endonucleases can be achieved by genetic modification, chemical inhibition, protein depletion of (among others) endonucleases like DFF40 (DNA fragmentation factor 40), ENODG (Endonuclease G), and DNASE1L3, while indirect mechanisms involve genetic modification, chemical inhibition, protein depletion, mutation, synthetic misregulation of (among others) upstream regulators of the above mentioned endonucleases like DFF45 (DNA fragmentation factor 45), Caspase 3, Caspase7, granzyme B, and AiF (apoptosis inducing factor).

Respective genetic modifications that can achieve inhibition of DNA fragmentation caused by programmed cell death, e.g., by way of one or more of the above mechanisms, encompass a mechanism, selected from the group consisting of
(a) knockout of one or more genes, selected from the group consisting of *CASP3* gene, *DFFB* gene, and *ENDOG* gene,
(b) expression of one or more RNA silencing elements directed against one or more genes, selected from the group consisting of *CASP3* gene, *DFFB* gene, and *ENDOG* gene,
(c) expression of one or more dominant negative mutants of caspase-3, DFF40 (DNA fragmentation factor 40), and/or mitochondrial endonuclease G,
(d) overexpression of wildtype ICAD, preferably strong overexpression of wildtype ICAD,
(e) expression of intrabodies and similar devices targeting Caspase-3, DFF40, and/or mitochondrial endonuclease G on the protein level,
(f) transcriptional silencing by directing dCas9 to promoters of Caspase-3, DFF40, and/or ENDOG,
(g) exon skipping via U7 smOpt on DFFB, CASP3 or ENDOG pre-mRNA, and
(h) inhibitory isoform shifting, e.g. of ICAD by reduction of ASF/SF2.

Respective means of gene knockout, the expression of RNA silencing elements, the expression of dominant negative mutants of specific proteins, the overexpression of e.g. wiltype ICAD, the expression of intrabodies, transcriptional silencing via dCas9, exon skipping via U7 smOpt, and inhibitory isoform shifting are not particularly limited and are known in the art. By way of example, gene knockout can be effected by way of the CRISPR/Cas system, Zinc-finger nucleases or transcription activator-like effector nucleases (TALENs), or designer homing endonucleases/meganucleases, as known in the art. Further, the expression of RNA silencing elements can e.g., include the expression of suitable microRNAs (miRNA) or small interfering RNAs (siRNA), as known in the art.

In a third aspect, the present invention relates to a method for producing AAV, comprising the step of producing AAV in a cell line as defined above for the first and second aspects of the present invention.

Methods for producing AAV in a given cell line are not particularly limited and are known in the art.

In a fourth aspect, the present invention relates to a method of producing Adeno-associated virus (AAV), comprising the step of exposing the cells in which AAV is produced to an inhibitor of DNA fragmentation caused by programmed cell death during the AAV production phase.

According to this aspect of the present invention, the inhibition of DNA fragmentation caused by programmed cell death is not effected endogenously, *i.e.,* by way of a genetic modification of the cells in which AAV is produced, but exogenously, *i.e.,* by way of exposing the cells in which AAV is produced to an inhibitor of DNA fragmentation caused by programmed cell death and/or an inhibitor of apoptotic, parthanatotic and/or pyroptotic pathways during the AAV production phase.

Chemical inhibitors of DNA fragmentation caused by programmed cell death are not particularly limited and are known in the art. However, in preferred embodiments, the inhibitor of DNA fragmentation caused by programmed cell death is selected from the group consisting of Z-VAD-fmk (Z-Val-Ala-Asp fluoromethyl ketone; CAS No. 161401-82-7); Z-IETD-fmk (Z-Ile-Glu-Thr-Asp; CAS. No. 210344-98-2); Z-DEVD-fmk (Z-Asp-Glu-Val-Asp; CAS. Nr. 210344-95-9); PNR-3-80 (5-((1-(2-naphthoyl)-5-chloro-1H-indol-3-yl)methylene)-2-thioxodihydro-pyrimidine-4,6(1H,5H)-dione); PNR-3-82 (5-((1-(2-naphthoyl)-5-methoxy-1H-indol-3-yl)methylene)-2-thioxodihydropyrimidine-4,6(1H,5H)-dione); Zn²⁺ ions; EDTA (ethylenediaminetetraacetic acid); and proteolysis-targeting chimeras (PROTACs) directed against caspase-3, DFF40 (DNA fragmentation factor 40), and/or mitochondrial endonuclease G, wherein Z-VAD-fmk is particularly preferred.

Respective chemical inhibitors can be added to the cells in an AAV production process at any time from five days prior to the start of AAV production until two days after start of AAV production. Suitable concentration ranges are not particularly limited and are known in the art. These include e.g. a range of 0.5 to 20 µg/ml, preferably of 10 to 20 µg/ml, for for Z-VAD-fmk, Z-IETD-fmk, or Z-DEVD-fmk.

In this context, PROTACs are heterobifunctional small molecules composed of two active domains and a linker, capable of removing specific unwanted proteins. PROTACs work by inducing selective intracellular proteolysis, and consist of two covalently linked protein-binding molecules, one capable of engaging an E3 ubiquitin ligase, and another that binds to a target protein meant for degradation. Recruitment of the E3 ligase to the target protein results in ubiquitination and subsequent degradation of the target protein via the proteasome.

As used herein, the term "comprising"/"comprises" expressly includes the terms "consisting essentially of"/"consists essentially of and "consisting of"/"consists of", *i.e.,* all of said terms are interchangeable with each other herein.

Further, as used herein, the term "about" preferably represents a modifier of the specified value of ± 10%, more preferably ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention has surprisingly identified DNA fragmentation caused by programmed cell death as the major source of nuclease-resistant product-related contamination by host cell DNA.

The present invention advantageously prevents or reduces the packaging of host cell DNA during the production of the AAV particles in the host cell and thus significantly reduces nuclease-resistant product-related contamination. Specifically, the present invention prevents or reduces nuclease-resistant contamination of AAV products while these are still being produced and without affecting the production of the AAV particles in the host cell, wherein by way of inhibiting DNA fragmentation caused by programmed cell death, the packaging of host cell DNA is reduced.

During the production of AAV particles in human host cells, the production process itself kills the host cell. This death occurs, among other things, through programmed cell death.

The packaging of wild-type AAV genomes occurs in the nucleus of infected cells and is mediated by the AAV Rep78 protein that bridges the Rep binding element (RBE) located on the vector genome-associated Inverted Terminal Repeats (ITRs) to sites proximal to pores on the surface of preformed AAV empty capsids, thus mediating the translocation of single-stranded DNA vector genomes into the preformed capsids. However, Rep78 expression results in the activation of caspase-3 and induces apoptotic cell death and disruption of the cell cycle.

Apoptosis is a form of programmed cell death that occurs in multicellular organisms and is governed by a set of cysteinyl-aspartate specific proteases (caspases) that can be triggered by intrinsic and extrinsic stimuli. Once activated, caspases execute the apoptotic cell death in an irreversible cascade. The main morphological changes of the apoptotic process include cell and nuclear shrinkage, chromatin condensation, internucleosomal cleavage of chromosomal DNA, formation of apoptotic bodies and phagocytosis by neighboring cells.

During apoptosis, host cell DNA is degraded by so-called endonucleases into defined pieces. The process of DNA fragmentation is controlled, among other things, by the cleavage of ICAD (inhibitor of caspase activated DNase) activated by caspase-3 and the subsequent activation of nuclease CAD (caspase activated DNase). The cleavage of genomic DNA by these endonucleases takes place specifically between the nucleosomes and produces DNA fragments with a length of about 180 base pairs and multiples thereof.

Apoptotic DNA fragmentation is mediated by DNA fragmentation factor (DFF) which is a heterodimeric protein composed of DFF 45 (ICAD) and DFF 40 (CAD). DFF40 contains an intrinsic DNase activity, while DFF45 serves as an inhibitor of DFF40 activity. Following activation of apoptosis, caspase-3 cleaves DFF45 and DFF45 dissociates from DFF40 which cleaves DNA into oligonucleosome sized fragments (180 bp intervals) known as "apoptotic ladder" when visualized by agarose gel electrophoresis.

Thus, the present invention advantageously reduces product-related impurities, affording increased product quality. Moreover, in doing so, the present invention improves viral genome packaging resulting in an increased percentage of full capsids. Furthermore, the present invention increases host cell viability, resulting in an increase of productivity.

The figures show:
Figure 1:
   Fragment length distribution of AAV encapsulated DNA that align to a) host cell DNA or b) rAAV genome (1800 bp).
   Y-axis represents the frequency of fragments, the x-axis the corresponding read length in bp.
Figure 2:
   Fragment length distribution of AAV encapsulated DNA that aligns to host cell DNA.
   Y-axis represents the frequency of fragments, the x-axis the corresponding read length in bp. Dashed lines represent the maximum of individual peaks within the fragment distribution. (peak1 = 181 bp, peak2 = 325 bp, peak3 = 507 bp, peak4 = 708 bp).
Figure 3:
   Viability of induced stable producer clone 1 and stable producer clone 2 at different concentrations of Z-VAD-fmk.
   a) Stable producer clone 1 (*upper panel*) and 2 (*lower panel*)*.* Relative viable cell density (solid diamonds) and viability (solid circles) measured at different time points post induction and supplementation [hours]. Z-VAD-fmk concentrations ranging from 10 µg/ml (darkest grey) over 2.5 µg/ml (dark grey) to 0 µg/ml (light grey).
   b) *Upper panel:* Relative viable cell density (grey bars) and viability (black dots) at 4 days post induction/transfection of a CAP Alpha cell line in a semi-transient AAV producer setting using different concentrations of Z-VAD-fmk. 1: no Z-VAD-fmk; 2: 2 µM: 3: 5 µM; 4: 10 µM; 5: 20 µM Z-VAD-fmk. *Lower panel:* a stable packaging pool without transfection of a cargo plasmid. Measured at 72 h post transfections. Viabilities indicated by black circles.1: no Z-VAD-fmk; 2: 10 µM Z-VAD-fmk; 3: 20 µM Z-VAD-fmk; 4: DMSO; 5: 10 µM Camptothecin; 6: 10 µM Camptothecin + 20 µM Z-VAD-fmk
Figure 4:
   Inhibition of apoptotic DNA fragmentation reduces amount and packaging of hcDNA.
   a) *upper row:* sensitivity test for Sub-G1 DNA-fragmentation assay (Chemometec) using Camptothecin treatment (10 µM) on parental CAP cells over night. M1 population as % of global DNA stain. *Lower row:* merged curves for apoptotic DNA fragmentation determined by same assay after 0 h (black curve), 18 h (dark grey), and 44 h (light grey), with Z-VAD-fmk (right) and in control samples (left, induction only)
   b) *Left:* Relative share of encapsulated hcDNA [%] in transient and stable AAV8 production at 3 days post induction (transient transfection) and 5 days post induction (stable clone). Odd-numbered bars: no Z-VAD-fmk; even-numbered bars: 10 µg/ml Z-VAD-fmk; 1-6: CAP cells with transient transfection: 1+2: Rep/Cap + cargo 1; 3+4: Rep/Cap + cargo 2; 5+6: Rep/Cap, no cargo. 7+8: HEK293 transiently transfected with Rep/Cap + cargo 1; 9+10: stable producer clone 1, no transfection; 11: Background assay control. Encapsulated hcDNA was measured in relation to the respective untreated controls, each set at 100%. *Right:* fold difference of hcDNA concentration over time [hours] in an untreated AAV production using stable producer clone 1 (light grey line) normalized to 10 µg/ml of Z-VAD-fmk (dark grey line) determined by ALU qPCR.
   c) *Left:* Relative Capsid levels determined by ELISA in stable producer clone 1 and transiently transfected HEK293 suspension cells at different Z-VAD-fmk concentrations and compared with Camptothecin. Samples harvested at 5 days post induction. 1: untreated control; 2: HEK293 + 20 µM Z-VAD-fmk; 3: HEK293 + 40 µM Z-VAD-fmk; 4: stable producer clone 1 + 20 µM Z-VAD-fmk; 5: stable producer clone 1 + 40 µM Z-VAD-fmk; 6: stable producer clone 1 + DMSO; 7: stable producer clone 1 + 10 µM Camptothecin. *Right:* corresponding host cell DNA per capsid as determined by ALU qPCR normalized to untreated controls. Relative shares indicated above bars. 1: untreated control; 2+3: HEK293; 2: 20 µM of Z-VAD-fmk; 3: 40 µM Z-VAD-fmk; 4-7: stable producer clone 1; 4: 20 µM Z-VAD-fmk; 5: 40 µM Z-VAD-fmk; 6: DMSO; 7: 10 µM Camptothecin.
Figure 5:
   Ratios of viral genome containing capsids upon treatment with Z-VAD-fmk.
   Influence of Z-VAD-fmk on semi-transient transfection of a stable packaging pool or full transient transfection of CAP cells. Vg/ml measured by qPCR and normalized to untreated samples (grey bars) and viral genomes normalized to amount of capsid determined by ELISA (black diamonds).
   Odd-numbered: untreated controls; Even-numbered: 20 µM Z-VAD-fmk; 1-4: AAV8 packaging pool; 1+2: cargo 1; 3+4: cargo 2; 5-8: CAP (full transient); 5+6: cargo 1, 7+8: cargo 2

The present invention relates to the following nucleotide sequences:
SEQ ID NO: 1
   SV40 PolyA Primer forward
   5'-AGCAATAGCATCACAAATTTCACAA-3'
SEQ ID NO: 2
   SV40 PolyA Primer reverse
   5'-CCAGACATGATAAGATACATTGATGAGTT-3'
SEQ ID NO: 3
   SV40 PolyA Probe FAM-ZEN-IBFQ
   5'-AGCATTTTTTTCACTGCATTCTAGTTGTGGTTTGTC-3'
SEQ ID NO: 4
   hcDNA Alu Primer forward
   5'-GAGGCGGGCGGATCA-3'
SEQ ID NO: 5
   hcDNA Alu Primer reverse
   5'-CCCGGCTAATTTTTGTATTTTTAGTAG-3'
SEQ ID NO: 6
   hcDNA Alu Probe HEX-ZEN-IBFQ
   5'-CAGCCTGGCCAACATGGTGAAACC-3'

The present invention will be further illustrated by the following example without being limited thereto.

### Examples

### Experimental procedures:

### Long-read sequencing using PacBio:

rAAV samples were treated with Denarase and purified by affinity chromatography.

PacBio Long-read sequencing was performed as known in the art. Briefly, encapsulated DNA was purified, DNA ends were repaired, and barcode adapters ligated. Damaged regions were repaired, the libraries were purified, and primers were annealed. Finally, the DNA polymerase was bound, and the DNA fragments were sequenced on the PacBio Sequel platform with v2.0 chemistry.

Bioinformatic Analysis Workflow:
∘ PacBio raw data were demultiplexed using PacBio SMRT Link
∘ Circular consensus sequencing (CCS) reads were generated by PacBio SMRT Link
∘ Alignment of CCS reads to reference using pbmm2
∘ Parsing of BAM files using samtools and custom scripts
∘ Counting and visualization of CCS reads was performed using R/Bioconductor

### Cell culture:

Four different types of producer cells were used:
1. Parental CAP or HEK293 cells, which carry adenoviral E1A and E1B genes and which were transiently transfected with genes encoding the AAV Cap proteins VP1, VP2, and VP3; genes encoding the AAV Rep proteins Rep78, Rep68, Rep52, and Rep40; genes encoding the adenoviral helper functions E4orf6, E2A and VA-RNA and the gene of interest (GOI) flanked by AAV inverted terminal repeat sequences (ITRs) for AAV production (fully transient approach).
2. "Alpha"-cells are derivatives of parental cells and harbor additional stably inserted and inducible genes encoding the AAV Rep proteins Rep78, Rep68, Rep52, and Rep40 and genes encoding the adenoviral helper functions E4orf6, E2A and VA-RNA. Upon transfection of a capsid-encoding plasmid alongside a cargo sequence, Alpha cells are enabled to produce AAVs (semi-transient system).
3. "Packaging" cells originate from Alpha cells and additionally carry stably integrated inducible genes for the AAV Cap proteins VP1, VP2, and VP3.
4. "Full producers", are derived from packaging cells and additionally carry the gene of interest (GOI) flanked by AAV inverted terminal repeat sequences (ITRs) thus having stably integrated all genes necessary to produce AAVs.

CAP cells were routinely cultivated in chemically defined, serum-free PEM medium (Thermo Fisher Scientific) supplemented with 4 mM GlutaMax (Gibco) in shake flasks (125 mL; Corning) on a shaking incubator at 120 rpm (5 cm orbit), 5 % CO₂ and 37 °C.

During routine cultivation, cells were diluted with fresh medium to a viable cell density of 0.5-1×10⁶ cells/ml every 72 to 96 h. Viable cell density and viability were determined by trypan blue exclusion using a Vi-Cell Blu cell counter (Beckman Coulter).

### Transient transfection

Transient transfection was performed using PEImax (PolySciences) and 1 pg plasmid per cell in FreeStyle F17 medium (Thermo Fisher Scientific) with 4 mM Glutamax and 50 µl/L IGF. AAV production was induced by adding doxycycline (Clontech) to a final concentration of 1 µg/mL. AAV8 serotype expression was under control of a doxycycline inducible promoter and cargo plasmids contained AAV2-ITRs flanking a GFP reporter construct. Plasmids were transfected at a 1:1 ratio. Stable producer cells were induced by addition of doxycycline without further transfection. Caspase inhibitor Z-VAD-fmk (InvivoGen) was added using the indicated concentrations at time point of induction.

At different time points post transfection, cell suspension was harvested, and cells were lysed by addition of Triton-X 100 (Sigma). After centrifugation, supernatants were diluted and incubated with TurboDNase (ThermoFisher Scientific), in order to remove non-encapsulated DNA contaminants. After inactivation of TurboDNAse, proteinase K was added to digest the viral capsid and subsequently amplify viral genomic DNA or hcDNA via ddPCR or qPCR.

### qPCR/ddPCR to determine viral titer and host cell DNA:

The following primer/dual-labelled probe combinations (ordered at MWG, Eurofins and IDT, respectively, Table 1) directed against
a) the SV40 PolyA (quantification of viral titer) or
b) Alu sequences (quantification of hcDNA)
   were used:
   As standard for the viral genome, linearized transgene plasmid with a defined copy number was used. As standard for the hcDNA assay previously isolated CAP DNA (purified with DNeasy^{®} Blood & Tissue Kit) was used.

**Table 1: Primer/Probe combination for qPCR and ddPCR assays**

| **Primer/Probe** | **Sequence** |
|---|---|
| SV40 PolyA Primer for (SEQ ID NO: 1) | 5'-AGCAATAGCATCACAAATTTCACAA-3' |
| SV40 PolyA Primer rev (SEQ ID NO: 2) | 5'-CCAGACATGATAAGATACATTGATGAGTT-3' |
| SV40 PolyA Probe FAM-ZEN-IBFQ (SEQ ID NO: 3) | 5'-AGCATTTTTTTCACTGCATTCTAGTTGTGGTTTGTC-3' |
| hcDNA_Alu_fwd (SEQ ID NO: 4) | 5'-GAGGCGGGCGGATCA-3' |
| hcDNA_Alu_rev (SEQ ID NO: 5) | 5'-CCCGGCTAATTTTTGTATTTTTAGTAG-3' |
| hcDNA_Alu_Probe HEX-ZEN-IBFQ (SEQ ID NO: 6) | 5'-CAGCCTGGCCAACATGGTGAAACC-3' |

AAV samples were treated with Turbo DNAse (Thermo Fischer Scientific) to digest the non-encapsulated DNA before the capsids were digested using Proteinase K. Proteinase K was inactivated by a heating step at 95°C for 15 min. The samples were measured either by qPCR or ddPCR as follows:
The qPCR reaction contained the following components: 2 x Brilliant Multiplex qPCR Master Mix (Agilent), nuclease-free H₂O (Thermo Fisher Scientific), primer/probe mix and sample/standard. qPCR was run on an LightCycler 480 according to the manufacturer's instructions.

In addition, ddPCR was used for absolute quantification of viral titer. Prior to droplet formation, samples were diluted in sample dilution buffer (GeneAmp 10x PCR Buffer I (Thermo Fischer Scientific), Sheared Salmon Sperm (SSS) DNA, Pluronic F-68 non-ionic surfactant) and added to the 2 × ddPCR Supermix (BioRad) with the appropriate primers/probes (cf. Table 1). Droplets were generated using the Biorad QX200 Droplet Generator according to the manufacturer's instructions. PCR was performed in the Biorad C100 Touch^{™} thermal cycler. Droplets were counted using the BioRad QX200 Droplet Reader and analyzed using QuantaSoft Analysis Software.

### DNA fragmentation assay

A DNA fragmentation assay by Chemometec was applied using the NucleoCounter^{®} NC-3000^{™} according to note No. 3003. Rev. 1.4.

In brief, cells were permeabilized with Ethanol, which allows low molecular weight DNA fragments (e.g., apoptotic DNA) to leak out, while large fragments and undamaged chromosomes are retained inside the nucleus. Total DNA content was stained by DAPI and detected using the NucleoCounter^{®} NC-3000^{™}. Here, cells typically fall into one of three clusters when plotted by signal intensity. The G1 peak represents the majority of cells with 2n DNA content. A second, smaller peak represents cells in G2 phase of the cell cycle, containing 4n. An intermediate spread-out population between the G1 and G2 peak represents cells in S-phase while cells with apparent sub-G1 DNA content are indicative of apoptotic DNA fragmentation and can be discerned due to ethanol-induced release of apoptotic fragments.

### Example 1:

### Analysis of nuclease-resistant DNA in recombinant AAV particles

### Introduction:

- The fragment length of DNA encapsulated into recombinant AAV particles mapping to a) host cell genome or b) to rAAV genome was determined. For this, purified and DNasel treated rAAV particles were analyzed using the PacBio technology.

### Result:

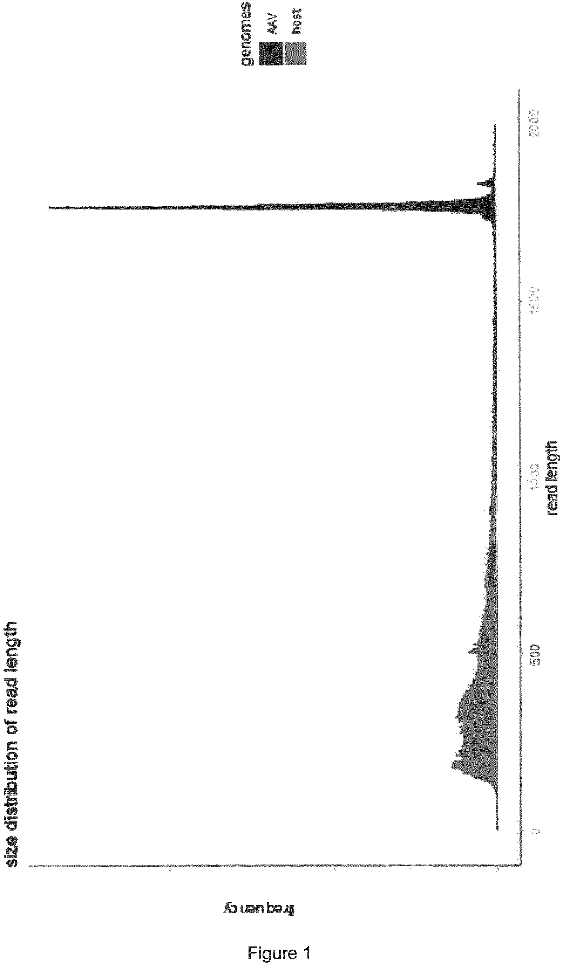

PacBio analysis revealed a distinct separation of DNA aligned to host cell genome and the rAAV genome in terms of fragment length. The rAAV genome showed an average fragment length of approximately 1800bp that matched with the actual size of the ITR flanked region. The size of DNA that maps to host cell genome showed a broader range starting at approximately 100bp (Fig. 1).

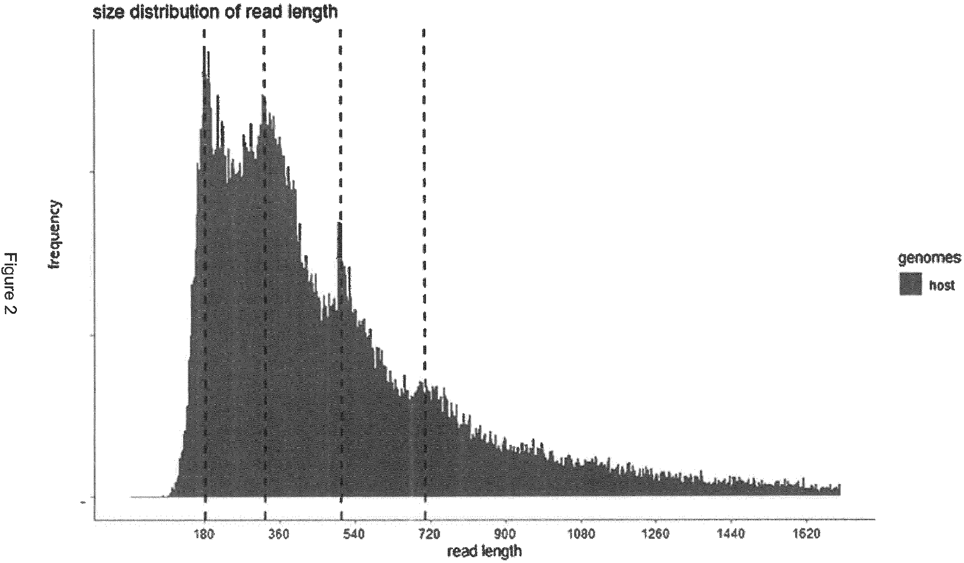

The detailed analysis of the host cell DNA fragment length in Fig. 2 revealed a wave-like pattern with distinct peaks. The maxima of each peak (visualized by dashed lines) matched to the size distribution of the theoretical size distribution of the apoptotic ladder with an averaged deviation of +/-20 bp as shown in Table 2.

**Table 2: Comparison of the theoretical DNA fragment length after apoptotic fragmentation and of the actual host cell DNA fragment length (calculated by peak maximas) present in the viral particle analyzed by PacBio.**

| **DNase protected nucleosomes** | **Apoptotic ladder [bp]** | **Peak maxima in PacBio seq [bp]** | **Deviation [bp]** |
|---|---|---|---|
| 1 | 180 | 181 | +1 |
| 2 | 360 | 325 | -35 |
| 3 | 540 | 507 | -33 |
| 4 | 720 | 708 | -12 |

These data lead to the assumption that the source of encapsulated host cell DNA in AAV productions is the fragmentation of DNA by the apoptotic pathway.

### Example 2:

Inhibition of apoptosis by pan-caspase inhibitor Z-VAD-fmk increases the relative number of viable cells after induction.

### Introduction:

- Various stable and transient AAV producer cells were induced with doxycycline and treated with different concentrations of the antiapoptotic pan-caspase inhibitor Z-VAD-fmk.
- Cell viability was evaluated using the NucleoCounter NC-3000.

### Result:

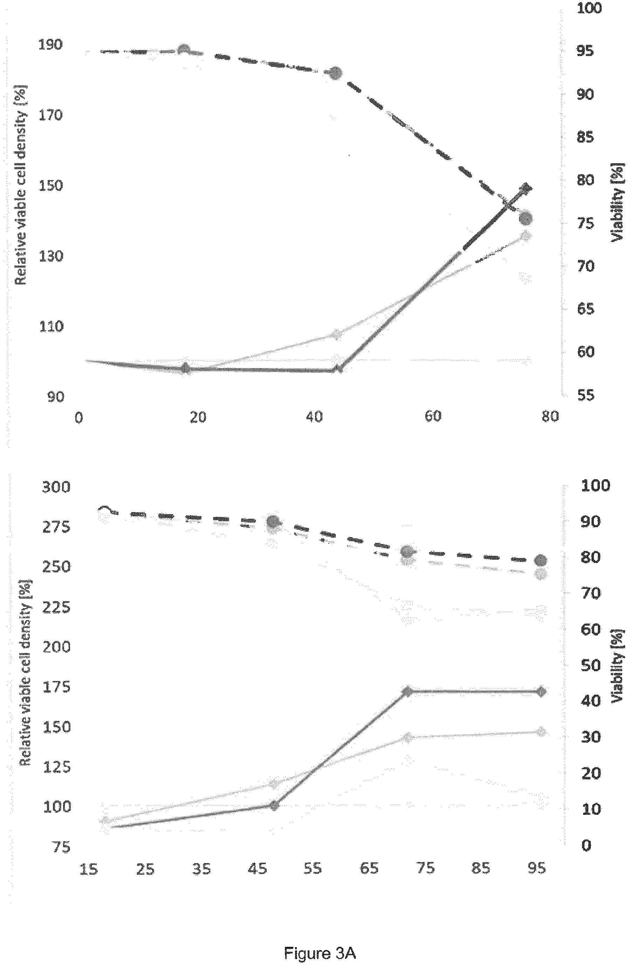

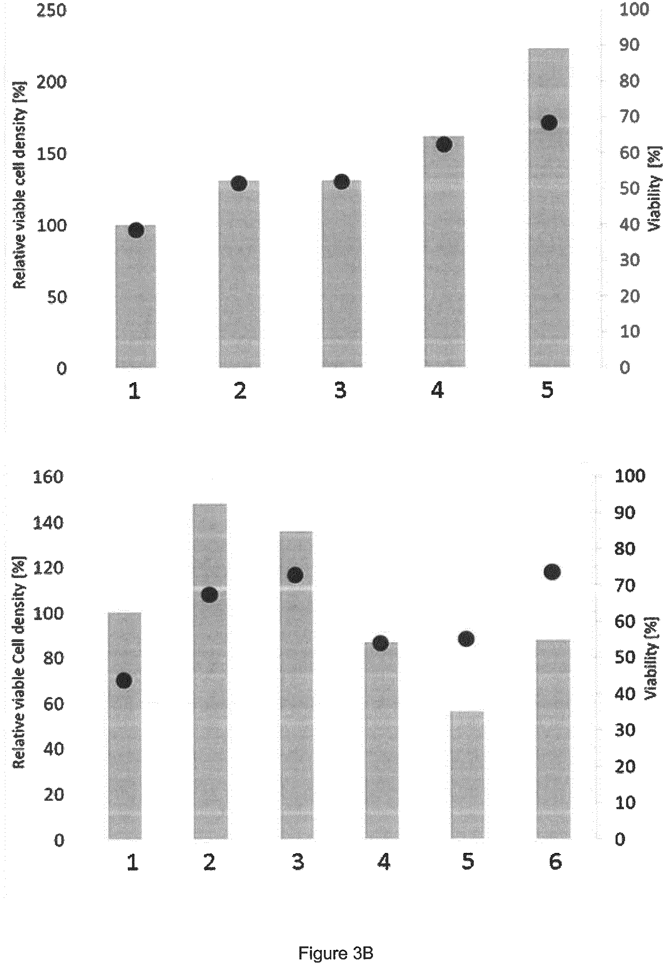

Treatment of cells with Z-VAD-fmk increased viable cell density and viability. The observed effects correlated with applied concentrations and increased viability by ~10 percentage points as compared to untreated cells over the course of 80 h of treatment with 10 µg/ml of Z-VAD-fmk (Fig. 3a) and independently from the investigated CAP cell line. In contrast, pro-apoptotic agent Camptothecin reduced viability and VCD (Fig. 3b), an effect that could be compensated by co-culturing with anti-apoptotic Z-VAD-fmk.

### Example 3:

Pan-caspase inhibitor Z-VAD-fmk drastically reduces amount of hcDNA in AAV particles.

### Introduction:

- Z-VAD-fmk inhibits caspases 3 and 8 which are upstream of the apoptotic nucleases CAD and ENDOG, respectively.
- Z-VAD-fmk treatment should therefore reduce chromosomal fragmentation and decrease amount of packageably sized AAV cargo.
- Transiently transfected CAP cells and HEK293 cells or full producers were cultured to produce AAV in the presence of absence of Z-VAD-fmk.
- Resulting AAV particles were harvested from cell suspension on days 1, 2, 3, and 4 post induction.
- Encapsulated host cell DNA was measured using a dedicated qPCR assay amplifying ALU repeats.
- DNA fragmentation was determined using the DNA fragmentation assay by Chemometec.

### Results:

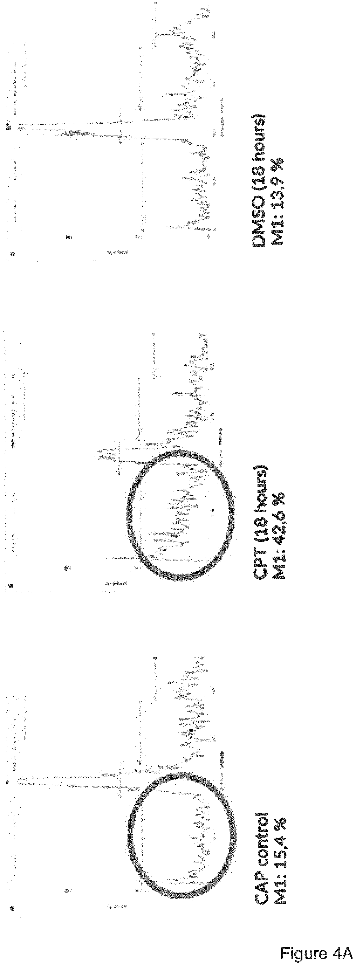

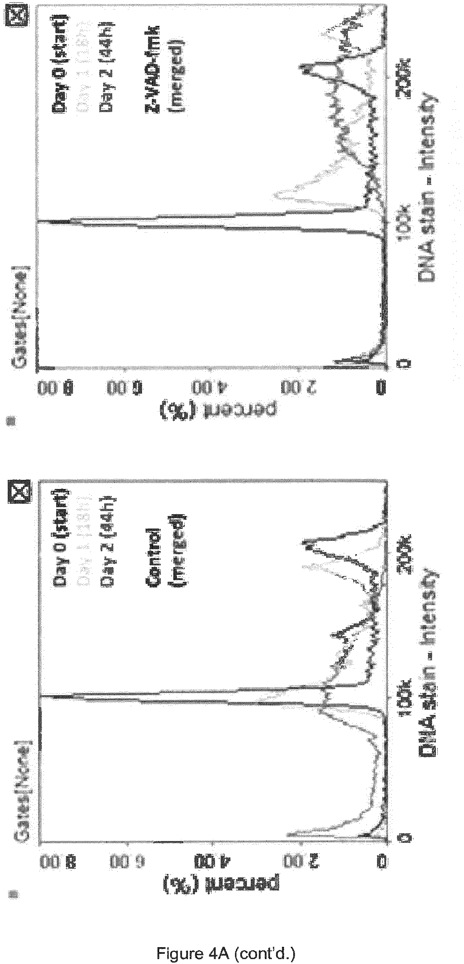

As predicted, Z-VAD-fmk reduced the amount of low molecular weight DNA that otherwise appears during vector production. Conversely, the analogous increase in short DNA fragments upon treatment with proapoptotic Camptothecin supports apoptotic origin of this type of DNA (Fig. 4A).

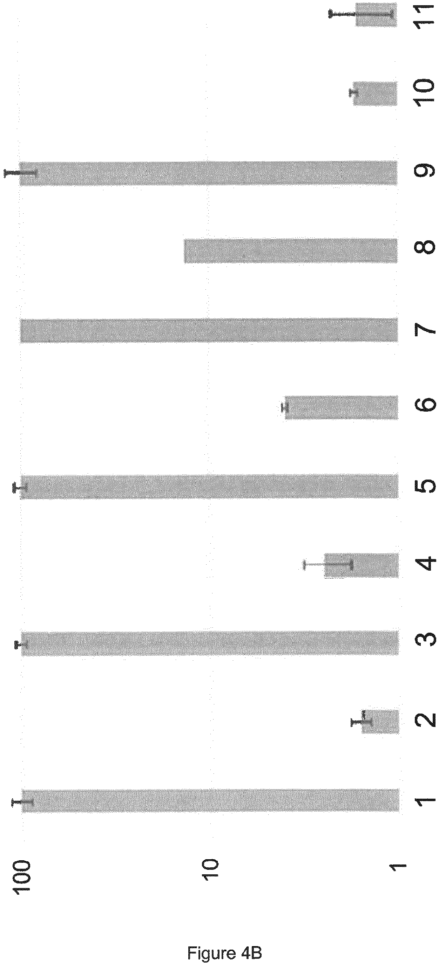

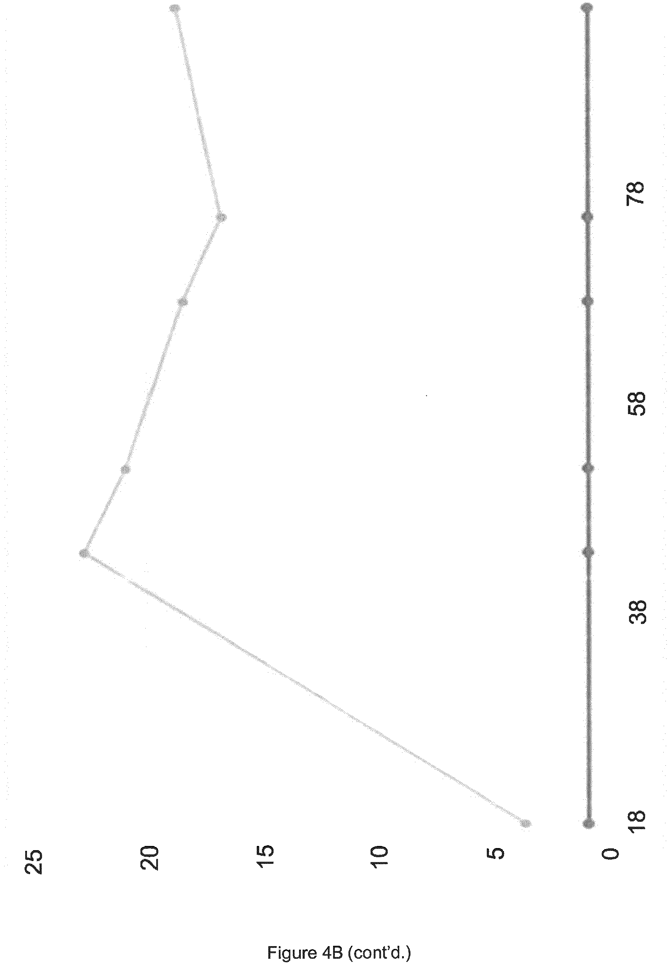

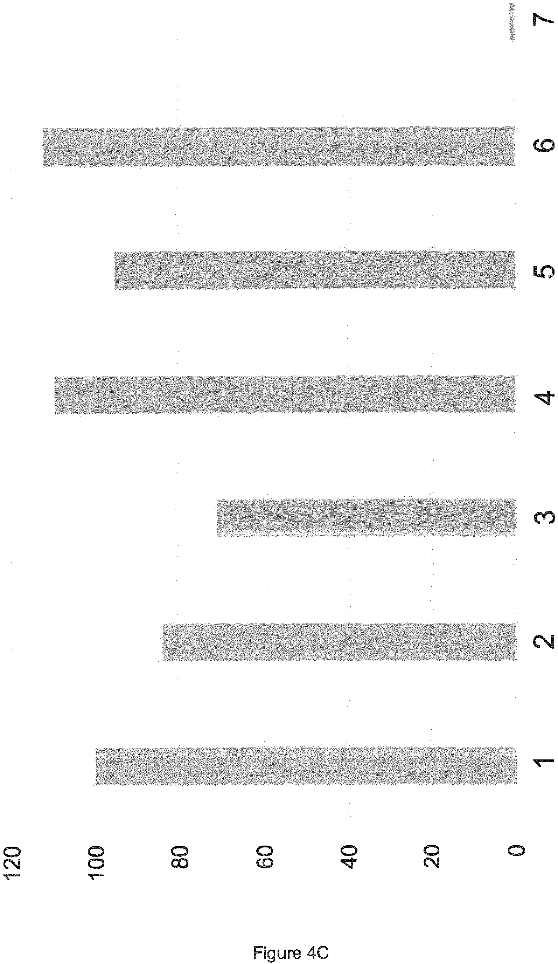

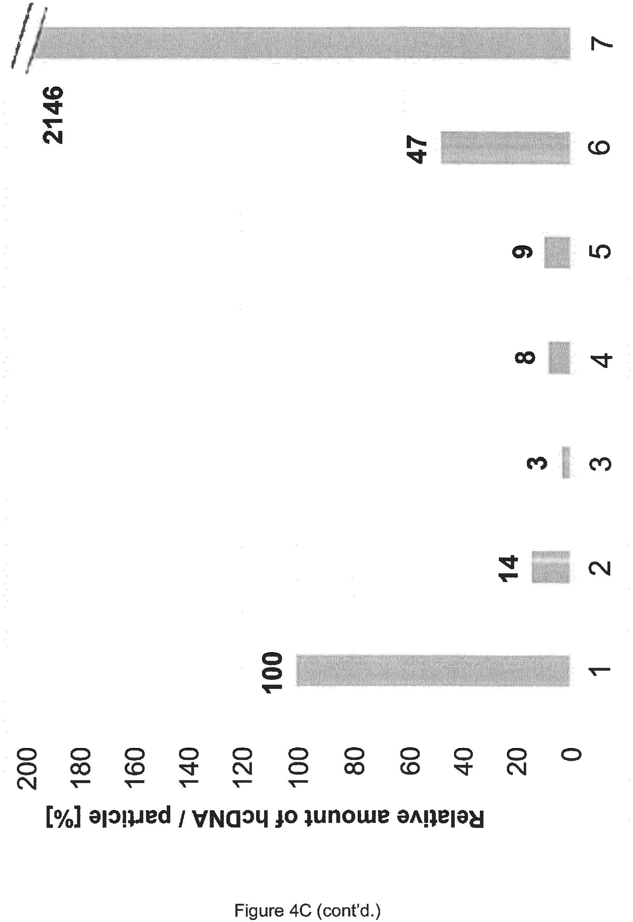

Analyses of viral particles revealed that treating cells with up to 40 µg/ml Z-VAD-fmk dramatically decreased packaging of host cell DNA compared to untreated controls (Figs. 4B and 4C). These results are consistently observed with the fully transient approach and full producers and are obtained with CAP as well as HEK293 cells.

ELISA readouts confirmed that reduced hcDNA levels are not the consequence of an overall decreased capsid production under Z-VAD-fmk treatment. On the contrary, in stable producer cells capsid levels were even slightly increased in the presence of Z-VAD-fmk and normalization to capsid levels (ELISA) revealed a ~10-fold reduction in encapsulated hcDNA encapsulated compared to controls (Fig. 4C).

### Example 4:

Pan-caspase inhibitor Z-VAD-fmk increases ratio of viral genome containing capsids to empty capsids.

### Introduction:

- Most recombinant AAV capsids remain devoid of viral genomes after assembly.
- Separation of full and empty particles is a major challenge in downstream processing of viral particles.
- Improving encapsulation of viral genomes would greatly reduce manufacturing costs and increase the quality of the final product.

### Results:

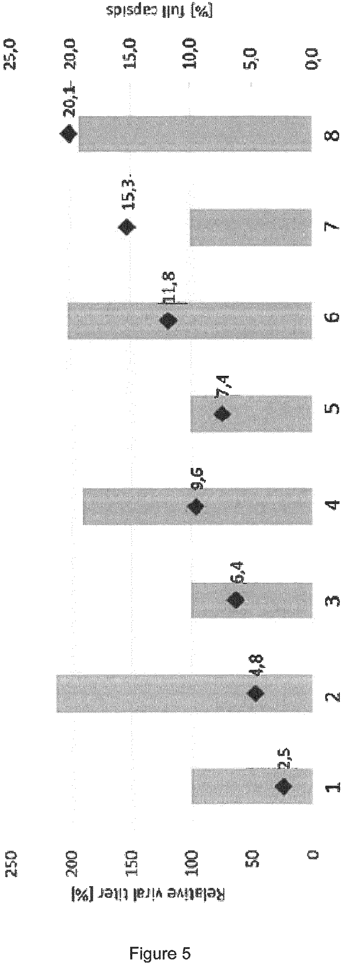

An AAV8 packaging pool and parental CAP cells were transfected with cargo plasmids and AAV production plasmids, respectively. Quantification of viral titers was performed using qPCR, capsid levels were determined using ELISA. Treatment with Z-VAD-fmk increased the proportion of viral genome-containing capsids by 30 to 90% in transient and semi-transient transfection approaches with two different cargo plasmids (Fig. 5).

## Claims

1. An Adeno-associated virus (AAV) producer cell line in which DNA fragmentation caused by programmed cell death is inhibited.

2. Use of a cell line in which DNA fragmentation caused by programmed cell death is inhibited for the production of Adeno-associated virus (AAV).

3. The use according to claim 2, wherein the cell line is an AAV producer cell line.

4. The AAV producer cell line according to claim 1, or the use according to claim 2 or claim 3, wherein the genes encoding the components necessary for the production of AAV are transiently expressed in said cell line or are stably integrated into the cell genome.

5. The AAV producer cell line or the use according to claim 4, wherein the genes encoding the components necessary for the production of AAV are selected from the group consisting of genes encoding the AAV Cap proteins VP1, VP2, and VP3; genes encoding the AAV Rep proteins Rep78, Rep68, Rep52, and Rep40; genes encoding the adenoviral helper functions E4orf6, E2A and VA-RNA; genes encoding the Ad5 helper genes E1A and E1B; and the gene of interest (GOI) flanked by AAV inverted terminal repeat sequences (ITRs).

6. The AAV producer cell line or the use according to claim 4 or claim 5, wherein the following genes are transiently expressed in said cell line or are stably integrated into the cell genome:
the genes encoding the AAV Cap proteins VP1, VP2, and VP3;
a gene or genes encoding either one or both of the AAV Rep proteins Rep78 or Rep68;
a gene or genes encoding either one or both of the AAV Rep proteins Rep52 or Rep40;
the gene of interest (GOI) flanked by AAV ITRs; and
optionally, a gene or genes encoding either one, either two, either three, or all of the adenoviral helper functions E1A, E1B, E4orf6 and E2A.

7. The AAV producer cell line according to any one of claims 1 to 6, or the use according to any one of claims 3 to 6, wherein the AAV producer cell line is derived from a cell line, selected from the group consisting of CAP cells, AGE1.hn, HEK293, PER.C6, NSO1, COS-7, BHK, CHO, Sf9, S2, CV1, VERO, HeLa, MDCK, BRL3A, W138, and HepG2 cells.

8. The AAV producer cell line according to any one of claims 1 to 7, or the use according to any one of claims 2 to 7, wherein DNA fragmentation caused by programmed cell death is apoptotic DNA fragmentation, parthanatotic DNA fragmentation, and/or pyroptotic DNA fragmentation.

9. The AAV producer cell line according to any one of claims 1 to 8, or the use according to any one of claims 2 to 8, wherein DNA fragmentation caused by programmed cell death is inhibited by way of direct or indirect inhibition of one or more endonucleases.

10. The AAV producer cell line or the use according to claim 9, wherein direct inhibition of one or more endonucleases encompasses
(i) genetic modification,
(ii) chemical inhibition, and/or
(iii) protein depletion,
of one or more endonucleases, selected from the group consisting of DFF40 (DNA fragmentation factor 40), ENODG (Endonuclease G), and DNASE1L3.

11. The AAV producer cell line or the use according to claim 9, wherein indirect inhibition of one or more endonucleases encompasses
(i) genetic modification,
(ii) chemical inhibition,
(iii) protein depletion,
(iv) mutation, and/or
(v) synthetic misregulation,
of one or more upstream regulators of one or more endonucleases, wherein said upstream regulators are selected from the group consisting of DFF45 (DNA fragmentation factor 45), caspase-3, caspase-7, granzyme B, and AiF (apoptosis inducing factor).

12. The AAV producer cell line according to any one of claims 1 to 11, or the use according to any one of claims 2 to 11, wherein DNA fragmentation caused by programmed cell death is inhibited by way of a mechanism, selected from the group consisting of
(a) knockout of one or more genes, selected from the group consisting of CASP3 gene, *DFFB* gene, and *ENDOG* gene,
(b) expression of one or more RNA silencing elements directed against one or more genes, selected from the group consisting of *CASP3* gene, *DFFB* gene, and *ENDOG* gene,
(c) expression of one or more dominant negative mutants of caspase-3, DFF40 (DNA fragmentation factor 40), and/or mitochondrial endonuclease G.
(d) overexpression of wildtype ICAD, preferably strong overexpression of wildtype ICAD,
(e) expression of intrabodies and similar devices targeting Caspase-3, DFF40, and/or mitochondrial endonuclease G on the protein level,
(f) transcriptional silencing by directing dCas9 to promoters of Caspase-3, DFF40, and/or ENDOG,
(g) exon skipping via U7 smOpt on DFFB, CASP3 or ENDOG pre-mRNA, and
(h) inhibitory isoform shifting, e.g. of ICAD by reduction of ASF/SF2.

13. A method for producing Adeno-associated virus (AAV), comprising the step of producing AAV in a cell line as defined in any one of claims 1 to 12.

14. A method of producing Adeno-associated virus (AAV), comprising the step of exposing the cells in which AAV is produced to an inhibitor of DNA fragmentation caused by programmed cell death during the AAV production phase.

15. The method according to claim 14, wherein the inhibitor of DNA fragmentation caused by programmed cell death is selected from the group consisting of Z-VAD-fmk (Z-Val-Ala-Asp fluoromethyl ketone; CAS No. 161401-82-7); Z-IETD-fmk (Z-Ile-Glu-Thr-Asp; CAS. No. 210344-98-2); Z-DEVD-fmk (Z-Asp-Glu-Val-Asp; CAS. Nr. 210344-95-9); PNR-3-80 (5-((1-(2-naphthoyl)-5-chloro-1H-indol-3-yl)methylene)-2-thioxodihydro-pyrimidine-4,6(1H,5H)-dione); PNR-3-82 (5-((1-(2-naphthoyl)-5-methoxy-1H-indol-3-yl)methylene)-2-thioxodihydropyrimidine-4,6(1H,5H)-dione); Zn²⁺ ions; EDTA (ethylenediaminetetraacetic acid); and proteolysis-targeting chimeras (PROTACs) directed against caspase-3, DFF40 (DNA fragmentation factor 40), and/or mitochondrial endonuclease G.
